(19) Europäisches Patentamt
European Patent Office
Office européen des brevets

(11) Veröffentlichungsnummer: **0 110 278**
**B1**

# (12) EUROPÄISCHE PATENTSCHRIFT

(45) Veröffentlichungstag der Patentschrift: **28.10.87**

(51) Int. Cl.⁴: **C 07 D 231/20**, C 08 K 5/34

(21) Anmeldenummer: **83111607.4**

(22) Anmeldetag: **21.11.83**

## (54) Pyrazolinone, ihre Herstellung und ihre Verwendung zur Stabilisierung von Polyvinylchlorid-Formmassen.

(30) Priorität: **29.11.82 DE 3244096**

(43) Veröffentlichungstag der Anmeldung: **13.06.84 Patentblatt 84/24**

(45) Bekanntmachung des Hinweises auf die Patenterteilung: **28.10.87 Patentblatt 87/44**

(84) Benannte Vertragsstaaten: **AT BE CH DE FR GB LI NL**

(56) Entgegenhaltungen: **US - A - 2 931 814**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Krause, Horst-Jürgen, Dr., Am Nettchesfeld 22, D-4000 Düsseldorf (DE)**
Erfinder: **Wegemund, Bernd, Dr., Händelweg 3, D-5657 Haan 1 (DE)**
Erfinder: **Erwied, Werner, Dr., Senliser Strasse 22, D-4018 Langenfeld (DE)**
Erfinder: **Krampitz, Dieter, Zeppelinstrasse 29, D-4050 Mönchengladbach 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Gegenstand der Erfindung sind neue Pyrazolinone der Formel I,

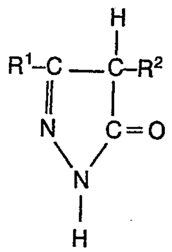

(I)

in der $R^1$ und $R^2$ geradkettige Alkylreste mit 6 bis 11 Kohlenstoffatomen bedeuten.

Die Erfindung betrifft weiterhin ein Verfahren zur Herstellung der Pyrazolinone der Formel I, bei dem man β-Ketocarbonsäureester der Formel IV,

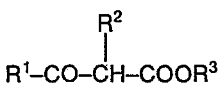

(IV)

in der $R^1$ und $R^2$ die für die Formel I angegebene Bedeutung haben und $R^3$ einen Alkylrest mit 1 bis 3 Kohlenstoffatomen bedeutet, mit Hydrazinhydrat umsetzt und das gewünschte Produkt aus dem Reaktionsgemisch isoliert.

Von den 3,4-Dialkyl-2-pyrazolin-5-onen der Formel I sind insbesondere diejenigen von Interesse, in denen $R^1$ einen Alkylrest der Formel $C_nH_{2n+1}$ (II) und $R^2$ einen Alkylrest der Formel $C_{n-1}H_{2n-1}$ (III) darstellt, wobei n die Werte 7, 9 und 11 annehmen kann.

Die als Ausgangsmaterial für die Herstellung der Pyrazolinone der Formel I benötigten β-Ketocarbonsäureester der Formel IV stellen bekannte Verbindungen dar, die nach gängigen Methoden der organischen Synthese erhalten werden können, beispielsweise durch Selbstkondensation von Fettsäureestern niederer Alkohole (Houben-Weyl: Methoden der organischen Chemie, 4. Auflage, Band 8, Stuttgart 1952, Seiten 567–570). Als besonders zweckmässig hat sich hier die Selbstkondensation von Fettsäuremethylestern unter der Einwirkung von Natriummethylat bei 100 bis 125 °C und laufender Entfernung des Methanols erwiesen, wobei die freien Ketocarbonsäuren durch Neutralisation mit Essigsäure bei 0 bis 20 °C aus den Esterenolaten erhalten werden.

Als β-Ketocarbonsäureester der Formel IV kommen insbesondere die aus den entsprechenden Estern der Caprylsäure, Caprinsäure und Laurinsäure erhältlichen Methyl-, Ethyl-, Propyl- und Isopropylester der 3-Oxo-2-hexyldecansäure, 3-Oxo-2-octyldodecansäure und 3-Oxo-2-decyltetradecansäure in Betracht.

Die Umsetzung der β-Ketocarbonsäureester der Formel IV mit Hydrazinhydrat wird zweckmässigerweise in einem polaren Lösungsmittel durchgeführt, z.B. in Methanol, Ethanol oder Isopropanol. Die Reaktion wird in der Regel mit stöchiometrischen Mengen β-Ketocarbonsäureester und Hydrazinhydrat durchgeführt. Man kann aber auch mit einem Überschuss des einen oder des anderen Reaktionspartners arbeiten, wenn dies im Hinblick auf eine höhere Ausbeute erforderlich erscheint.

Bei der praktischen Durchführung des erfindungsgemässen Herstellungsverfahrens ist es zweckmässig, den gesamten β-Ketocarbonsäureester in Form einer Lösung vorzulegen und die vorgesehene Menge Hydrazinhydrat – ebenfalls als Lösung in dem gewählten Lösungsmittel – unter Rühren langsam zuzugeben.

Die Reaktion verläuft spontan und exotherm. Es ist deshalb angezeigt, die entstehende Reaktionswärme durch Kühlung abzuführen. Es hat sich als zweckmässig erwiesen, während der Zugabe des Hydrazinhydrats die Temperatur des Reaktionsgemisches im Bereich von 40 bis 80 °C zu halten. Nach beendeter Hydrazinhydratzugabe kann das Reaktionsgemisch zur Vervollständigung der Reaktion einige Zeit, beispielsweise 1 bis 3 Stunden zum Rückflusskochen erhitzt werden.

Der grösste Teil des bei der Reaktion entstandenen Pyrazolinons fällt in der Regel beim Abkühlen des Reaktionsgemisches auf 0 °C in kristalliner Form aus und kann durch Filtration isoliert werden. In manchen Fällen scheidet sich das Pyrazolinon als ölige Flüssigkeit ab, die nach dem Abtrennen beim längeren Stehenlassen durchkristallisiert. Weitere Anteile des gewünschten Pyrazolinons können durch Einengen des Filtrats und erneutes Abkühlen erhalten werden.

Die auf diese Weise hergestellten 3,4-Dialkyl-2-pyrazolin-5-one können durch Umkristallisieren aus geeigneten Lösungsmitteln, beispielsweise aus den bereits erwähnten niederen Alkoholen, gereinigt werden, sofern dies erforderlich erscheint.

Ein weiterer Aspekt der Erfindung ist die Verwendung der Pyrazolinone der Formel I zur Stabilisierung von thermoplastischen Formmassen auf Basis von Polyvinylchlorid oder im wesentlich Vinylchlorid enthaltenden Mischpolymerisaten. Die Erfindung erstreckt sich hier sowohl auf Polyvinylchlorid-Formmassen mit einem Gehalt an Pyrazolinonen der Formel I als auch auf Stabilisatorkombinationen, die solche Pyrazolinone enthalten.

Bei der formgebenden Verarbeitung von thermoplastischen Polymerisaten zu Rohren, Flaschen, Profilen, Folien und dergleichen, beispielsweise durch Extrudieren, Spritzgiessen, Blasen, Tiefziehen und Kalandrieren, kann bei den dabei auftretenden hohen Temperaturen leicht ein Abbau des Kunststoffs eintreten. Dies führt zu unerwünschten Verfärbungen und zu einer Minderung der mechanischen Eigenschaften. Aus diesem Grund setzt man den Polymerisaten vor der Warmverformung Stabilisatoren zu, die einem solchen Abbau entgegenwirken. Für Polyvinylchlorid und im wesentlichen Vinylchlorid enthaltende Mischpolymerisate setzt man als Wärmestabilisatoren vor allem anorganische und organische Bleisalze, organische Antimonverbindungen, Organozinnverbindungen sowie Cadmium-/Bariumcarboxylate und -phenolate ein. Die genannten Metallverbindungen werden gewöhnlich als Primärstabilisatoren bezeichnet; zur Verbesserung ihrer Wirksamkeit setzt man ihnen häufig Sekundär- oder Costabilisatoren zu. Weitere Einzelheiten über die für Vinylchloridpolymerisate gebräuchlichen Wärmestabilisatoren können der einschlägigen Literatur entnommen werden, beispielsweise der Encyclopedia of Polymer Science and Technology, Band 12, New York, London, Sydney, Toronto 1970, Seiten 737 bis 768.

Die genannten Stabilisatoren zeigen in der Praxis eine durchaus befriedigende Wirkung. Gegen den Einsatz von Blei-, Antimon- und Cadmiumverbindungen bestehen jedoch gewisse Bedenken, insbesondere solche toxikologischer Art. Eine Reihe von Organozinnverbindungen ist toxikologisch unbedenklich, ihrem breiten Einsatz steht jedoch ihr hoher Preis entgegen. Aus diesen Gründen sucht man schon seit langem, diese Verbindungen durch weniger bedenkliche und preisgünstigere Substanzen zu ersetzen. In diesem Zusammenhang ist man dazu übergegangen, als Primärstabilisatoren fettsaure Salze, aromatische Carboxylate und Phenolate der Metalle Calcium, Barium, Zink und Aluminium zu verwenden, die gegebenenfalls durch Costabilisatoren wie z.B. organische Phosphite, Iminoverbindungen, Epoxyverbindungen, mehrwertige Alkohole oder 1,3-Diketone ergänzt werden. Diese neueren Stabilisatorsysteme sind jedoch in mehr oder minder grossem Masse mit dem Mangel behaftet, dass sie den zu stabilisierenden Formmassen eine zu geringe Anfangsstabilität und/oder eine zu geringe Langzeitstabilität verleihen. Es besteht daher ein Bedürfnis nach Substanzen, mit deren Hilfe sich die Anfangs- und/oder Langzeitwirkung derartiger Stabilisatorsysteme entscheidend verbessern lässt.

Es wurde gefunden, dass sich die 3,4-Dialkyl-2-pyrazolin-5-one der Formel I mit Erfolg zur Stabilisierung von Polyvinylchlorid-Formmassen verwenden lassen. Dabei wurde insbesondere festgestellt, dass die Verbindungen der Formel I in der Lage sind, die stabilisierende Wirkung von Primärstabilisatoren auf Basis von Seifen, aromatischen Carboxylaten und Phenolaten der Metalle Calcium, Barium, Zink und Aluminium sowie von Organozinnverbindungen in nichtvorhersehbarem Masse zu erhöhen.

Gegenstand der Erfindung sind demnach auch stabilisierte Polyvinylchlorid-Formmassen, die Pyrazolinone der Formel I enthalten, insbesondere solche Polyvinylchlorid-Formmassen, die neben üblichen Gleitmitteln und anderen üblichen Verarbeitungshilfsmitteln eine Stabilisatorkombination aus (1) Primärstabilisatoren ausgewählt aus der aus Metallseifen, aromatischen Metallcarboxylaten, Metallphenolaten und Organozinnverbindungen bestehenden Gruppe und (2) Pyrazolinone der Formel I als Costabilisatoren enthalten.

Weiterhin sind Gegenstand der Erfindung Stabilisatorkombinationen für Polyvinylchlorid-Formmassen, die Primärstabilisatoren ausgewählt aus der aus Metallseifen, aromatischen Metallcarboxylaten, Metallphenolaten und Organozinnverbindungen bestehenden Gruppe und Pyrazolinone der Formel I als Costabilisatoren und gegebenenfalls übliche Gleitmittel und andere übliche Verarbeitungshilfsmittel enthalten.

In bevorzugten Ausführungsformen enthalten die Polyvinylchlorid-Formmassen bzw. die Stabilisatorkombinationen Pyrazolinone der Formel I, in der $R^1$ einen Alkylrest der Formel $C_nH_{2n+1}$ (II) und $R^2$ einen Alkylrest der Formel $C_{n-1}H_{2n-1}$ (III) bedeuten, wobei n die Werte 7, 9 und 11 annehmen kann.

Unter Metallseifen werden im Zusammenhang mit der Erfindung fettsaure Salze des Calciums, Bariums, Zinks und Aluminiums verstanden. Diese Metallseifen leiten sich vorzugsweise von Fettsäuren mit 8 bis 22 Kohlenstoffatomen ab. Die Fettsäurekomponente kann dabei insbesondere aus Capryl-, Caprin-, Laurin-, Myristin-, Palmitin-, Stearin- und Behensäure bestehen. Darüber hinaus kommen verzweigtkettige Fettsäuren wie 2-Ethylhexansäure, 2-Hexyldecansäure und 2-Octyldodecansäure und Hydroxyfettsäuren, beispielsweise 9,(10)-Hydroxystearinsäure und 9,10-Dihydroxystearinsäure, in Betracht. Die erfindungsgemässen Zusammensetzungen können sowohl Salze einzelner Fettsäuren als auch Salze von Fettsäuregemischen enthalten, wie sie aus natürlichen Fetten und Ölen gewonnen werden.

Als aromatische Metallcarboxylate kommen vor allem die Calcium-, Barium-, Zink- und Aluminiumsalze der Benzoesäure und substituierter, insbesondere alkylsubstituierter Benzoesäure in Betracht.

Als Metallphenolate kommen die Phenolate, Alkylphenolate und Naphthenate des Calciums, Bariums, Zinks und Aluminiums in Betracht. Auch sogenannte überbasische Phenolate oder Naphthenate sind brauchbare Zusätze.

Als Organozinnverbindungen kommen vor allem die Dialkylverbindungen des vierwertigen Zinns in Betracht, beispielsweise
Dimethylzinn-S,S-bis-(isooctylthioglykolat),
Dibutylzinndilaurat, Dibutylzinnmaleinat,
Dibutylzinn-bis-(monobutylmaleinat),
Dibutylzinn-bis-(monooctylmaleinat),
Dibutylzinn-bis-(laurylmercaptid),
Dibutylzinn-β-mercaptopropionat,
Dibutylzinn-S,S-bis-(isooctylthioglykolat),
Di-n-octylzinnmaleinat,
Di-n-octylzinn-bis-(monoethylmaleinat),
Di-n-octylzinn-β-mercaptopropionat und
Di-n-octylzinn-S,S-bis-(isooctylthioglykolat).

Die stabilisierten Polyvinylchlorid-Formmassen enthalten in der Regel auf 100 Gewichtsteile Polymerisat 0,02 bis 5 Gewichtsteile Pyrazolinon der Formel I. Die Metallseifen, aromatischen Metallcarboxylate, Metallphenolate und Organozinnverbindungen können in Mengen von 0,05 bis 5 Gewichtsteilen pro 100 Gewichtsteile Polymerisat anwesend sein.

In einer speziellen Ausführungsform der Erfindung enthalten die Polyvinylchlorid-Formmassen auf 100 Gewichtsteile Polymerisat 0,1 bis 3 Gewichtsteile Calciumseife und/oder 0,1 bis 3 Gewichtsteile Bariumseife und/oder 0,1 bis 3 Gewichtsteile Zinkseife, wobei sich die Seifen vorzugsweise von Fettsäuren mit 8 bis 22 Kohlenstoffatomen ableiten und der Gesamtanteil der Metallseifen normalerweise 3 Gewichtsteile pro 100 Gewichtsteile Polymerisat nicht übersteigt.

In vielen Fällen kann es günstig sein, den Polyvinylchlorid-Formmassen auf 100 Gewichtsteile Polymerisat 0,2 bis 5 Gewichtsteile eines synthetischen, kristallinen, 13 bis 25 Gewichtsprozent gebundenes Wasser enthaltenden, feinteiligen Natriumalumosilikats zuzusetzen, das – bezogen auf die wasserfreie Form – die Zusammensetzung 0,7 bis 1,1 $Na_2O \cdot Al_2O_3 \cdot 1,3–2,4\ SiO_2$ hat.

Bei den oben definierten Natriumalumosilikaten handelt es sich um Zeolithe vom Typ NaA, die einen durchschnittlich wirksamen Porendurchmesser von 4 Å besitzen, weshalb sie auch als Zeolithe 4 A bezeichnet werden. Die Verwendung dieser Zeolithe als Hilfsmittel bei der Verarbeitung von Thermoplasten ist bekannt, siehe beispielsweise US-PS 4 000 100 und EP-A-0 027 588.

Neben den genannten Zusätzen können die erfindungsgemässen Polyvinylchlorid-Formmassen Partialester aus Polyolen mit 2 bis 6 Kohlenstoffatomen und 2 bis 6 Hydroxylgruppen und Fettsäuren mit 8 bis 22 Kohlenstoffatomen als Gleitmittel mit stabilisierender Wirkung enthalten. Diese Partialester besitzen pro Molekül im Durchschnitt mindestens eine freie Polyol-Hydroxylgruppe und können durch Veresterung entsprechender Polyole mit Fettsäuren der angegebenen Kettenlänge – gegebenenfalls in Gegenwart von üblichen Veresterungskatalysatoren – hergestellt werden. Polyole und Fettsäuren werden dabei im Molverhältnis 1:1 bis 1:(n – 1) miteinander umgesetzt, wobei n die Anzahl der Hydroxylgruppen des Polyols bedeutet. Vorzugsweise setzt man die Komponenten in solchen Mengen ein, dass sich Partialester mit einer OH-Zahl zwischen 140 und 580, insbesondere zwischen 170 und 540 bilden. Das Reaktionsprodukt, das jeweils ein Estergemisch darstellt, soll eine Säurezahl unter 15, vorzugsweise unter 8, haben. Geeignete Polyolkomponenten sind

Ethylenglykol, Propylenglykol-1,2,
Propylenglykol-1,3, Butylenglykol-1,2,
Butylenglykol-1,4, Hexandiol-1,6,
Neopentylglykol, Trimethylolethan, Erythrit,
Mannit und Sorbit,
sowie insbesondere
Glycerin, Trimethylolpropan,
Ditrimethylolpropan, Pentaerythrit und
Dipentaerythrit.

Als geeignete Fettsäurekomponenten seien beispielsweise Capryl-, Caprin-, Laurin-, Myristin-, Palmitin-, Stearin- und Behensäure genannt. Es können auch synthetische Fettsäuren der erwähnten Kohlenstoffzahl oder ungesättigte Fettsäuren, wie Ölsäure und Linolensäure, oder auch substituierte Fettsäuren, insbesondere hydroxylierte Säuren wie 12-Hydroxystearinsäure eingesetzt werden. Aus praktischen Gründen setzt man zumeist Gemische von Fettsäuren ein, wie sie aus natürlichen Fetten und Ölen gewonnen werden.

Die erfindungsgemässen Polyvinylchlorid-Formmassen enthalten die Polyolpartialester in der Regel in Mengen von 0,2 bis 2,0 Gewichtsteilen auf 100 Gewichtsteile Polymerisat.

Es kann auch von Vorteil sein, wenn die erfindungsgemässen Polyvinylchlorid-Formmassen Thioglykolsäureester von Alkoholen mit 1 bis 6 Hydroxylgruppen und 3 bis 36 Kohlenstoffatomen enthalten. Hier kommen Thioglykolsäureester mit relativ geringer Flüchtigkeit in Betracht, beispielsweise Thioglykolsäureester des Glycerins, Trimethylolpropans, der isomeren Octanole, des Decanols, der Guerbetalkohole aus Octanolen, oder auch technischer dimerisierter Fettalkohole. Diese Ester können auch freie Hydroxylgruppen aufweisen; ebenso ist es möglich, Gemische aus Thioglykolsäureestern und freien mehrwertigen Alkoholen einzusetzen.

Die erfindungsgemässen Polyvinylchlorid-Formmassen können die genannten Thioglykolsäureester in Mengen von 0,1 bis 10 Gewichtsteilen auf 100 Gewichtsteile Polymerisat enthalten.

Weiterhin kann gegebenenfalls eine günstige Wirkung erzielt werden, wenn in den erfindungsgemässen Polyvinylchlorid-Formmassen 1,3-Dicarbonylverbindungen vorhanden sind. Im allgemeinen kommen solche 1,3-Diketone in Frage, die wenigstens zwei Alkyl-, Aryl- oder Aralkylreste, beispielsweise Dodecyl-, Hexadecyl-, Octadecyl-, Phenyl- oder Benzylreste im Molekül enthalten. Typische Vertreter dieser Substanzklasse sind Palmitoylstearoylmethan oder Stearoylbenzoylmethan.

Die erfindungsgemässen Polyvinylchlorid-Formmassen können solche 1,3-Dicarbonylverbindungen in Mengen von 0,1 bis 5 Gewichtsteilen auf 100 Gewichtsteile Polymerisat enthalten.

Die erfindungsgemässen Polyvinylchlorid-Formmassen können weitere Hilfsstoffe und Verarbeitungshilfsmittel enthalten, beispielsweise wachsartige Kohlenwasserstoffe wie Paraffine mit einem Erstarrungspunkt im Bereich von 45 bis 90 °C und/oder niedermolekulare Polyethylensorten, deren Erweichungspunkt unterhalb von 140 °C liegen soll. Zweckmässigerweise werden diese wachsartigen Substanzen mit freien Fettsäuren kombiniert, wobei Fettsäuren mit 12 bis 22 Kohlenstoffatomen, wie sie aus natürlichen Fetten und Ölen zugänglich sind, bevorzugt werden. Besonders günstige Ergebnisse werden mit Palmitin- und Stearinsäure erhalten. Auch handelsübliche Fettalkohole mit 12 bis 22 Kohlenstoffatomen können in den erfindungsgemässen thermoplastischen Formmassen enthalten sein.

Als weitere Additive kommen bestimmte Polymerisate in Betracht, beispielsweise solche auf Basis von Methylmethacrylat, Methylmethacrylat/Butylacrylat, Ethylacrylat, Methylmethacrylat/Acrylsäure und Butylmethacrylat/Styrol. Derartige Polymerisate und Copolymerisate werden als flow promoter bezeichnet. Polymerisate aus Butylacrylat wirken bei der formgebenden Verarbeitung von Polyvinylchlorid-Formmassen auch als Trennmittel.

In bestimmten Fällen kann es von Vorteil sein, wenn die erfindungsgemässen Polyvinylchlorid-Formmassen Antioxydationsmittel enthalten. Hier kommen beispielsweise
Diphenylolpropan,
2,5-Bis-(1,1-dimethylpropyl)-hydrochinon,
2,6-Di-tert.-butyl-4-methylphenol,
Octadecyl-(3,5-Di-tert.-butyl-4-hydroxyphenyl)-propionat,
1,1,3-tris-(5-tert.-butyl-4-hydroxy-2-methyl-phenyl)-butan oder
Dilaurylthiodipropionat
in Betracht.

Polyvinylchlorid-Formmassen, die für die Herstellung von Hohlkörpern und Folien bestimmt sind, können auf 100 Gewichtsteile Polymerisat 0,5 bis 5 Gewichtsteile epoxydiertes Sojaöl und 0,1 bis 0,8 Gewichtsteile höhermolekulares Esterwachs enthalten. Als hochmolekulare Esterwachse kommen Montanwachse, Paraffinoxydate und Komplexester in Betracht.

Die Basis der erfindungsgemässen stabilisierten thermoplastischen Formmassen besteht aus Homopolymerisaten oder Mischpolymerisaten des Vinylchlorids. Die Mischpolymerisate enthalten wenigstens 50 Molprozent, vorzugsweise 80 Molprozent Vinylchlorid. Die Polymerisate können auf beliebigem Wege, beispielsweise durch Suspensions-, Emulsions- oder Blockpolymerisaten hergestellt worden sein. Ihr K-Wert kann zwischen etwa 35 und 80 liegen. In den Rahmen der Erfindung fallen auch Formmassen auf Basis von nachchloriertem Polyvinylchlorid, sowie solche auf Basis von Harzgemischen, die überwiegend Homopolymerisate oder Copolymerisate des Vinylchlorids enthalten. Der Begriff Polyvinylchlorid-Formmassen erfasst erfindungsgemäss sowohl die zur Verformung bestimmten Halbfertigprodukte als auch aus diesen Massen beliebig geformte Artikel.

Die einzelnen Komponenten der erfindungsgemässen Polyvinylchlorid-Formmassen können durch Vermischen der Bestandteile in konventionellen Mischern vereinigt werden. Es ist dabei möglich, zunächst die verschiedenen Komponenten der Stabilisatorkombination miteinander zu vermischen und dieses Gemisch dann mit dem Polyvinylchlorid-Basismaterial zu vereinigen.

Die erfindungsgemässen Stabilisatorkombinationen für Polyvinylchlorid-Formmassen bestehen im einfachsten Fall aus einem Primärstabilisator ausgewählt aus der Gruppe bestehend aus Metallseifen, aromatischen Metallcarboxylaten, Metallphenolaten und Organozinnverbindungen und einem Pyrazolinon der Formel I.

Eine spezielle Ausführungsform der Erfindung sieht Stabilisatorkombinationen vor, in denen auf 1 Gewichtsteil Pyrazolinon der Formel I 0,5 bis 10 Gewichtsteile Calciumseifen und/oder 0,5 bis 10 Gewichtsteile Bariumseifen und/oder 0,5 bis 10 Gewichtsteile Zinkseifen vorhanden sind, wobei sich die Seifen von Fettsäuren mit 8 bis 22 Kohlenstoffatomen ableiten. Der Gesamtanteil der genannten Metallseifen soll dabei 20 Gewichtsteile pro 1 Gewichtsteil Pyrazolinon nicht überschreiten.

Als weitere Grundkomponente der erfindungsgemässen Stabilisatorkombinationen kommen die bereits weiter oben beschriebenen Natriumalumosilikate in Betracht, die in Mengen von 0,2 bis 20 Gewichtsteilen auf 1 Gewichtsteil Pyrazolinon der Formel I vorhanden sein können.

Bevorzugt enthalten die Stabilisatorkombinationen wenigstens ein Gleitmittel aus der Gruppe der oben definierten Partialester aus Fettsäuren und Polyolen. Dabei kann der Partialester in Mengen von 0,2 bis 5 Gewichtsteile auf 1 Gewichtsteil Pyrazolinon der Formel I vorhanden sein.

Die erfindungsgemässen Stabilisatorkombinationen können gewünschtenfalls durch weitere Hilfsstoffe und Verarbeitungshilfsmittel für Polyvinylchlorid-Formmassen ergänzt werden.

Die Stabilisatorkombinationen können durch einfaches mechanisches Vermischen der Bestandteile mit Hilfe von konventionellen Mischern erhalten werden. Bei der Herstellung fallen sie im allgemeinen als rieselfähige, nichtstaubende Produkte an.

Beispiele

I. Herstellung der Pyrazolinone der Formel I

Beispiel 1

Zu einer Lösung von 68,0 g (0,2 Mol) 3-Oxo-2-octyldodecansäuremethylester (2-Caprinoylcaprinsäuremethylester) in 200 ml Methanol wurde unter Rühren eine Lösung von 10,0 g (0,2 Mol) Hydrazinhydrat in 50 ml Methanol so langsam zugetropft, dass das exotherm reagierende Gemisch mittels äusserer Kühlung auf einer Temperatur von etwa 50 °C gehalten werden konnte. Nach dem Ende der Zugabe wurde das Gemisch zur Vervollständigung der Reaktion 2 Stunden lang zum Rückflusskochen erhitzt. Die Reaktionsmischung wurde auf 0 °C abgekühlt, wobei das Pyrazolinon auskristallisierte. Die Kristalle wurden abfiltriert, mit kaltem Methanol gewaschen und getrocknet. Es wurden 52 g (80,7% d.Th.) 3-Nonyl-4-octyl-2-pyrazolin-5-on mit Schmelzpunkt 69–71 °C erhalten. Durch Umkristallisieren aus Methanol wurde das Produkt analysenrein (Schmelzpunkt 71–72 °C) erhalten.

Analyse:
$C_{20}H_{38}N_2O$ (322,52)
berechnet (%): 74,48 C; 11,88 H; 8,69 N.
gefunden (%): 74,8 C; 12,1 H; 8,7 N.
IR: 1605 $cm^{-1}$(C = O)

Beispiel 2

Analog Beispiel 1 wurden 79,3 g (0,2 Mol) 3-Oxo-2-decyltetradecansäuremethylester (2-Lauroyllaurinsäuremethylester) in 200 ml Methanol mit 10,0 g (0,2 Mol) Hydrazinhydrat in 50 ml Methanol miteinander umgesetzt. Das rohe Pyrazolinon wurde aus Methanol umkristallisiert. Es wurden 60,0 g (79,2% d.Th.) 3-Undecyl-4-decyl-2-pyrazolin-5-on mit Schmelzpunkt 77–78 °C erhalten.

Analyse:
$C_{24}H_{46}N_2O$ (378,6)
berechnet (%): 76,13 C; 12,25 H; 7,40 N.

gefunden (%): 76,8 C; 12,6 H; 7,56 N.
IR: 1605 $cm^{-1}$ (C = O)

Beispiel 3

Zu einer Lösung von 569,0 g (2,0 Mol) 3-Oxo-2-hexyldecansäuremethylester (2-Capryloylcaprylsäuremethylester) in 500 ml Isopropanol wurde unter Rühren eine Mischung aus 100 g (2,0 Mol) Hydrazinhydrat in 100 ml Isopropanol in solchen Portionen zugetropft, dass das Gemisch mittels äusserer Kühlung auf 70–75 °C gehalten werden konnte. Nach dem Ende der Zugabe wurde das Gemisch 2 Stunden lang zum Rückflusskochen erhitzt. Nach dem Abkühlen auf etwa 30 °C wurden 200 ml Isopropanol und dann Wasser bis zur Trübung der Lösung zugegeben. Beim Abkühlen auf 0 °C kristallisierte das Pyrazolinon aus. Die Kristalle wurden abfiltriert, mit kaltem Isopropanol gewaschen und getrocknet. Es wurden 497 g (93% d. Th.) 3-Heptyl-4-hexyl-2-pyrazolin-5-on mit Schmelzpunkt 57–60 °C erhalten. Durch Umkristallisieren aus Aceton/Wasser wurde das Produkt analysenrein (Schmelzpunkt 61–62 °C) erhalten.

Analyse:
$C_{16}H_{30}N_2O$ (266,42)
berechnet (%): 72,13 C; 11,35 H; 10,52 N.
gefunden (%): 72,3 C; 11,7 H; 10,5 N.
IR: 1605 $cm^{-1}$ (C = O)

II. Verwendung der Pyrazolinone der Formel I zur Stabilisierung von Polyvinylchlorid-Formmassen

Herstellung und Prüfung der Walzfelle

In den Beispielen 4 bis 11 wurde die Wirkung der Stabilisatorkombinationen anhand der «statischen Thermostabilität» von Walzfellen geprüft. Zu diesem Zweck wurden Stabilisatorgemische enthaltende Polyvinylchlorid-Formmassen auf einem Laborwalzwerk der Abmessung 350 × 150 mm (Fa. Schwabenthan) bei einer Walzentemperatur von 170 °C und einer Walzendrehzahl von 30 Upm im Gleichlauf im Verlauf von 5 Minuten zu Prüffellen verarbeitet. Die ca. 0,5 mm dicken Felle wurden zu quadratischen Probestükken mit 10 mm Kantenlänge zerschnitten, die anschliessend in einem Trockenschrank mit 6 rotierenden Horden (Heraeus FT 420 R) einer Temperatur von 180 °C ausgesetzt wurden. Im Abstand von 10 Minuten wurden Proben entnommen und deren Farbveränderung begutachtet.

In der nachfolgenden Tabelle 1 ist in Abhängigkeit von der eingesetzten Stabilisatormischung die Zeit angegeben, nach der der Test wegen zu starker Verfärbung (Stabilitätsabbruch) beendet wurde.

Beispiel 4

100 GT (GT = Gewichtsteile) Suspensions-PVC (K-Wert 70; Vestolit S 7054®; Hersteller: Chemische Werke Hüls AG, Marl) wurden mit 0,5 GT 3-Heptyl-4-hexyl-2-pyrazolin-2-on (Beispiel 3) vermischt. Die so erhaltene thermoplastische Formmasse A wurde nach der oben angegebenen Methode geprüft. Der Stabilitätsabbruch (starke Verfärbung nach dunkelbraun/schwarz) trat nach 20 Minuten Prüfdauer ein.

Vergleichsversuch:

Wurde in der Formmasse A das 3-Heptyl-4-hexyl-2-pyrazolin-5-on weggelassen, so trat bei sonst gleicher Verfahrensweise schon bei der Herstellung der Walzfelle eine rotbraune Färbung auf.

Beispiel 5

Die thermoplastische Formmasse B wurde durch mechanisches Vermischen von

100 GT Suspensions-PVC (K-Wert 68; Vestolit® S 6858; Hersteller: Chemische Werke Hüls, Marl)
0,25 GT Zink-2-ethylhexanoat
1,0 GT 3-Nonyl-4-octyl-2-pyrazolin-5-on (Beispiel 1)

hergestellt. Bei der Prüfung der statischen Thermostabilität nach der oben angegebenen Methode wurde eine erste Verfärbung nach 20 Minuten beobachtet. Das Stabilitätsende (starke Verfärbung nach dunkelbraun/schwarz) trat nach 35 Minuten Prüfdauer ein.

Vergleichsversuch:

Wurde in der Formmasse B das 3-Nonyl-4-octyl-2-pyrazolin-5-on weggelassen, so trat bei sonst gleicher Verfahrensweise bereits zu Beginn der Walzfellherstellung eine starke Verfärbung nach schwarz (Stabilitätsabbruch) auf.

Beispiel 6

Durch mechanisches Vermischen der Komponenten wurde die Verarbeitungshilfsmittelkombination C′ hergestellt (GT = Gewichtsteile):

10 GT Calciumstearat
2 GT Stearinsäure
2 GT Paraffin
5 GT Pentaerythritester der Stearinsäure (Molverhältnis 1:1,5; OH-Zahl 212)
5 GT 3-Heptyl-4-hexyl-2-pyrazolin-5-on

Zur Herstellung der PVC-Formmasse C wurden

100 GT Suspensions-PVC (K-Wert 70; Vestolit® S 7054, Hersteller: Chemische Werke Hüls, Marl)
2,4 GT Kombination C′

miteinander vermischt. Bei der Prüfung der erhaltenen Formmasse nach der oben angegebenen Methode trat der Stabilitätsabbruch (starke Verfärbung nach dunkelbraun/schwarz) nach 30 Minuten Prüfdauer ein.

Vergleichsversuch:

Wurde in der Kombination C′ das 3-Heptyl-4-hexyl-2-pyrazolin-5-on weggelassen, so trat bei sonst gleicher Verfahrensweise schon bei der Herstellung der Walzfelle eine unerwünschte rotbraune Färbung auf.

Beispiel 7

Durch Vermischen von jeweils 100 GT Suspensions-PVC (K-Wert 70, siehe Beispiel 6) mit 2,4 GT

einer Verarbeitungshilfsmittelkombination der Zusammensetzung

10 GT Calciumstearat
5 GT Zinkstearat
2 GT Stearinsäure
2 GT Paraffin
5 GT Pentaerythritester der Stearinsäure (Molverhältnis 1:1,5; OH-Zahl 212)

wurden die PVC-Formmassen D, E, F und G hergestellt. Als weitere Zusätze wurden auf 100 GT Polymerisat eingearbeitet

in D: 0,2 GT 3-Nonyl-4-octyl-2-pyrazolin-5-on
in E: 0,5 GT 3-Nonyl-4-octyl-2-pyrazolin-5-on
in F: 1 GT 3-Nonyl-4-octyl-2-pyrazolin-5-on
in G: 0,5 GT 3-Heptyl-4-hexyl-2-pyrazolin-5-on und 1,0 GT synthetischer Zeolith NaA ($Na_2O:Al_2O_3:SiO_2$ = 0,9:1:2,4; Wassergehalt 19 Gewichtsprozent)

Die auf diese Weise erhaltenen PVC-Formmassen wurden nach der oben angegebenen Methode untersucht; die gefundenen Werte für die statische Thermostabilität sind in der Tabelle 1 wiedergegeben.

Tabelle 1

| Formmassen | erste Verfärbung (min) | Stabilitätsabbruch (min) |
|---|---|---|
| D | 10 | 30 |
| E | 10 | 40 |
| F | 10 | 50 |
| G | 10 | 70 |

Beispiel 8

Durch mechanisches Vermischen der Komponenten wurde die Stabilisatorkombination H' mit folgender Zusammensetzung hergestellt:

4 GT Calciumstearat
15 GT Isotridecylstearat
3 GT 3-Heptyl-4-hexyl-2-pyrazolin-5-on

Zur Herstellung der PVC-Formmasse H wurden

100 GT Emulsions-PVC (Vorstabilisiert mit Soda, Vestolit® E 7004, Hersteller: Chemische Werke Hüls, Marl)
20 GT Di-2-ethylhexylphthalat
2,2 GT Stabilisatorkombination H'

miteinander vermischt. Bei der Prüfung der erhaltenen Formmassen nach der oben angegebenen Methode trat der Stabilitätsabbruch nach 50 Minuten Prüfdauer ein.

Vergleichsversuch:

Wurde das 3-Heptyl-4-hexyl-2-pyrazolin-5-on weggelassen, so trat bei sonst gleicher Verfahrensweise bereits bei der Herstellung der Walzfelle eine unerwünschte rotbraune Färbung auf.

Beispiel 9

Beispiel 8 wurde wiederholt mit dem Unterschied, dass das mit Soda vorstabilisierte Emulsions-PVC durch das Suspensions-PVC aus Beispiel 6 ersetzt wurde. Der Stabilitätsabbruch wurde nach 40 Minuten Prüfdauer beobachtet.

Vergleichsversuch:

Wurde das 3-Heptyl-4-hexyl-2-pyrazolin-5-on weggelassen, so trat bei sonst gleicher Verfahrensweise bereits bei der Herstellung der Walzfelle eine unerwünschte Rosa-Färbung auf.

Beispiel 10

Durch mechanisches Vermischen der Komponenten wurde die Stabilisatorkombination I' der folgenden Zusammensetzung hergestellt:
2 GT Zinkstearat
6 GT Bariumstearat
1 GT 3-Heptyl-4-hexyl-2-pyrazolin-5-on

Zur Herstellung der PVC-Formmasse I wurden
100 GT Suspensions-PVC (K-Wert 70, Vinnol® H 70 F, Hersteller: Wacker Chemie, München)
50 GT Di-2-ethylhexylphthalat
2 GT epoxydiertes Sojaöl
0,9 GT Stabilisatorkombination I'
miteinander vermischt. Die erhaltene Formmasse wurde nach der oben angegebenen Methode geprüft. Der Stabilitätsabbruch trat nach 70 Minuten Prüfdauer ein.

Vergleichsversuch:

Wurde das 3-Heptyl-4-hexyl-2-pyrazolin-5-on weggelassen, so trat bei sonst gleicher Verfahrensweise bereits nach 40 Minuten Prüfdauer der Stabilitätsabbruch ein.

Beispiel 11

Die thermoplastische PVC-Formmasse J wurde durch mechanisches Vermischen von 100 GT Suspensions-PVC (K-Wert 70; Vinnol® H 70 F; Hersteller: Wacker-Chemie GmbH, München), 50 GT Di-2-ethylhexylphthalat und 0,95 GT der Stabilisatorkombination J' der Zusammensetzung
32 GT Bariumbenzoat
13 GT Zink-p-tert.-butylbenzoat
40 GT Tris-laurylphosphit
10 GT 3-Heptyl-4-hexyl-2-pyrazolin-5-on
erhalten. Die erhaltene Formmasse wurde nach der angegebenen Methode auf ihre statische Thermostabilität geprüft. Das Auftreten der ersten Verfärbung wurde dabei nach 40 Minuten beobachtet. Der Stabilitätsabbruch trat nach 60 Minuten Prüfdauer ein.

Vergleichsversuch:

Wurde in der Stabilisatormischung J' das 3-Heptyl-4-hexyl-2-pyrazolin-5-on weggelassen, so trat bei sonst gleicher Verfahrensweise bereits nach 30 Minuten Prüfdauer diese erste Verfärbung auf. Das Stabilitätsende wurde nach 40 Minuten Prüfdauer erreicht.

**Patentansprüche**

1. Pyrazolinone der Formel I

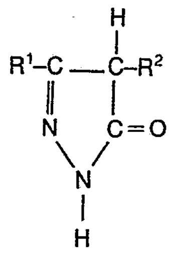

(I)

in der $R^1$ und $R^2$ geradkettige Alkylreste mit 6 bis 11 Kohlenstoffatomen bedeuten.

2. Pyrazolinone nach Anspruch 1, wobei in der Formel I $R^1$ einen Alkylrest der Formel $C_{n-1}H_{2n+1}$ (II) und $R^2$ einen Alkylrest der Formel $C_{n-1}H_{2n-1}$ (III) bedeutet und n die Werte 7, 9 und 11 annehmen kann.

3. Verfahren zur Herstellung von Pyrazolinonen der Formel I

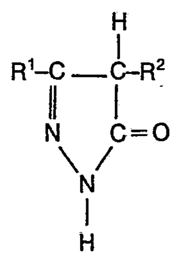

(I)

in der $R^1$ und $R^2$ geradkettige Alkylreste mit 6 bis 11 Kohlenstoffatomen bedeuten, bei dem man β-Ketocarbonsäureester der Formel IV

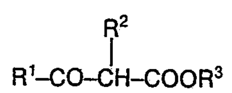

(IV)

in der $R^1$ und $R^2$ geradkettige Alkylreste mit 6 bis 11 Kohlenstoffatomen darstellen und $R^3$ einen Alkylrest mit 1 bis 3 Kohlenstoffatomen bedeutet, mit Hydrazinhydrat umsetzt und das gewünschte Produkt aus dem Reaktionsgemisch isoliert.

4. Verfahren nach Anspruch 3, dadurch gekennzeichnet, dass in der Formel IV $R^1$ einen Alkylrest der Formel $C_nH_{2n+1}$ (II) und $R^2$ einen Alkylrest der Formel $C_{n-1}H_{2n-1}$ (III) bedeuten, wobei n die Werte 7, 9 und 11 annehmen kann.

5. Stabilisierte Polyvinylchlorid-Formmassen enthaltend Pyrazolinone der Formel I

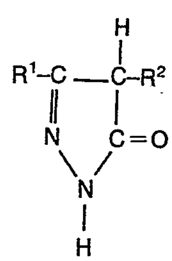

(I)

in der $R^1$ und $R^2$ geradkettige Alkylreste mit 6 bis 11 Kohlenstoffatomen bedeuten.

6. Stabilisierte Polyvinylchlorid-Formmassen nach Anspruch 5 enthaltend übliche Gleitmittel und andere übliche Verarbeitungshilfsmittel sowie eine Stabilisatorkombination aus (1) Primärstabilisatoren ausgewählt aus der Gruppe bestehend aus Metallseifen, aromatischen Metallcarboxylaten, Metallphenolaten und Organozinnverbindungen und (2) Costabilisatoren, dadurch gekennzeichnet, dass sie Pyrazolinone der Formel I nach Anspruch 1 als Costabilisatoren enthalten.

7. Stabilisierte Polyvinylchlorid-Formmassen nach den Ansprüchen 5 und 6, dadurch gekennzeichnet, dass sie auf 100 Gewichtsteile Polymerisat 0,02 bis 5 Gewichtsteile Pyrazolinon enthalten.

8. Stabilisierte Polyvinylchlorid-Formmassen nach den Ansprüchen 5 bis 7, dadurch gekennzeichnet, dass sie Pyrazolinone der Formel I enthalten, in der $R^1$ einen Alkylrest der Formel $C_nH_{2n+1}$ (II) und $R^2$ einen Alkylrest der Formel $C_{n-1}H_{2n-1}$ (III) bedeuten, wobei n die Werte 7, 9 und 11 annehmen kann.

9. Stabilisierte Polyvinylchlorid-Formmassen nach den Ansprüchen 5 bis 8, dadurch gekennzeichnet, dass sie auf 100 Gewichtsteile Polymerisat 0,1 bis 3,0 Gewichtsteile Calciumseifen und/oder 0,1 bis 3,0 Gewichtsteile Bariumseifen und/oder 0,1 bis 3,0 Gewichtsteile Zinkseifen enthalten, wobei sich die Seifen von Fettsäuren mit 8 bis 22 Kohlenstoffatomen ableiten.

10. Stabilisierte Polyvinylchlorid-Formmassen nach den Ansprüchen 5 bis 9, dadurch gekennzeichnet, dass sie auf 100 Gewichtsteile Polymerisat 0,2 bis 5 Gewichtsteile eines synthetischen kristallinen, 13 bis 25 Gewichtsprozent gebundenes Wasser enthaltenden, feinteiligen Natriumalumosilikats enthalten, das – bezogen auf die wasserfreie Form – die Zusammensetzung 0,7–1,1 $Na_2O{\cdot}Al_2O_3{\cdot}$1,3–2,4 $SiO_2$ hat.

11. Stabilisatorkombination für Polyvinylchlorid-Formmassen, enthaltend Primärstabilisatoren ausgewählt aus der Gruppe bestehend aus Metallseifen, aromatischen Metallcarboxylaten, Metallphenolaten und Organozinnverbindungen, Costabilisatoren und gegebenenfalls übliche Gleitmittel und andere übliche Verarbeitungshilfsmittel, dadurch gekennzeichnet, dass sie als Costabilisatoren Pyrazolinone der Formel I

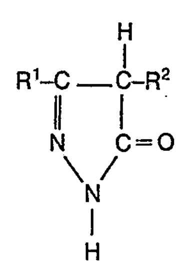

(I)

in der $R^1$ und $R^2$ geradkettige Alkylreste mit 6 bis 11 Kohlenstoffatomen bedeuten, enthalten.

12. Stabilisatorkombination nach Anspruch 11, dadurch gekennzeichnet, dass sie Pyrazolinone der Formel I enthalten, in der $R^1$ einen Alkylrest der Formel $C_nH_{2n+1}$ (II) und $R^2$ einen Alkylrest der Formel $C_{n-1}H_{2n-1}$ (III) bedeuten, wobei n die Werte 7, 9 und 11 annehmen kann.

13. Stabilisatorkombination nach den Ansprüchen 11 und 12, dadurch gekennzeichnet, dass sie auf 1 Gewichtsteil Pyrazolinon der Formel I 0,5 bis 10 Gewichtsteile Calciumseifen und/oder 0,5 bis 10 Gewichtsteile Bariumseifen und/oder 0,5 bis 10 Gewichtsteile Zinkseifen enthalten, wobei sich die Seifen von Fettsäuren mit 8 bis 22 Kohlenstoffatomen ableiten.

14. Stabilisatorkombination nach den Ansprüchen 11 und 12, dadurch gekennzeichnet, dass sie auf 1 Gewichtsteil Pyrazolinon der Formel I 0,2 bis 20 Gewichtsteile eines syntheytischen, kristallinen, 13 bis 25 Gewichtsprozent gebundenes Wasser enthaltenden, feinteiligen Natriumalumosilikats enthält, das – bezogen auf die wasserfreie Form – die Zusammensetzung 0,7–1,1 $Na_2O \cdot Al_2O_3 \cdot 1{,}3–2{,}4\ SiO_2$ hat.

**Claims**

1. Pyrazolinones corresponding to the following formula

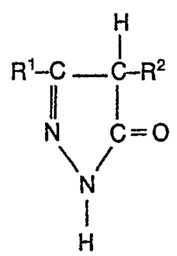

(I)

in which

$R^1$ and $R^2$ are linear $C_6$–$C_{11}$ alkyl radicals.

2. Pyrazolinones as claimed in Claim 1, wherein $R^1$ in formula I is an alkyl radical of the formula $C_{n-1}H_{2n+1}$ (II) and $R^2$ is an alkyl radical of the formula $C_{n-1}H_{2n-1}$ (III) and n may assume the values 7, 9 and 11.

3. A process for the production of pyrazolinones corresponding to the following formula

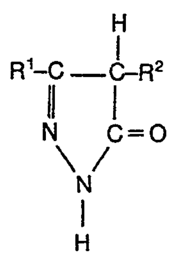

(I)

in which

$R^1$ and $R^2$ are linear $C_6$–$C_{11}$ alkyl radicals, wherein β-ketocarboxylic acid esters corresponding to the formula

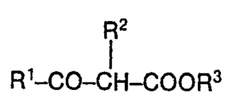

(IV)

in which

$R^1$ and $R^2$ are linear $C_6$–$C_{11}$ alkyl radicals and $R^3$ is a $C_1$–$C_3$ alkyl radical,

are reacted with hydrazine hydrate and the desired product is isolated from the reaction mixture.

4. A process as claimed in Claim 3, characterized in that $R^1$ in formula IV is an alkyl radical of the formula $C_nH_{2n+1}$ (II) and $R^2$ is an alkyl radical of the formula $C_{n-1}H_{2n-1}$ (III), in which formulae n may assume the values 7, 9 and 11.

5. Stabilized polyvinyl chloride molding compositions containing pyrazolinones corresponding to the following formula

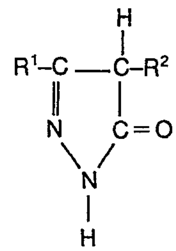

(I)

in which

$R^1$ and $R^2$ are linear $C_6$–$C_{11}$ alkyl radicals.

6. Stabilized polyvinyl chloride molding compositions as claimed in Claim 5 containing standard lubricants and other standard processing aids and also a stabilizer combination of (1) primary stabilizers selected from the group comprising metal soaps, aromatic metal carboxylates, metal phenolates and organotin compounds and (2) costabilizers, characterized in that they contain pyrazolinones of formula I according to Claim 1 as costabilizers.

7. Stabilized polyvinyl chloride molding compositions as claimed in Claims 5 and 6, characterized in that they contain from 0.02 to 5 parts by weight pyrazolinone to 100 parts by weight polymer.

8. Stabilized polyvinyl chloride molding compositions as claimed in Claims 5 to 7, characterized in that they contain pyrazolinones of formula I in which $R^1$ is an alkyl radical of the formula $C_nH_{2n+1}$ (II) and $R^2$ is an alkyl radical of the formula $C_{n-1}H_{2n-1}$ (III), in which formulae n may assume the values 7, 9 and 11.

9. Stabilized polyvinyl chloride molding compositions as claimed in Claims 5 to 8, characterized in that they contain from 0.1 to 3.0 parts by weight calcium soaps and/or from 0.1 to 3.0 parts by weight barium soaps and/or from 0.1 to 3.0 parts by weight zinc soaps to 100 parts by weight polymer, the soaps being derived from $C_8$–$C_{22}$ fatty acids.

10. Stabilized polyvinyl chloride molding compositions as claimed in Claims 5 to 9, characterized in that they contain from 0.2 to 5 parts by weight of a synthetic, crystalline, finely divided sodium alumosilicate containing from 13 to 25% by weight bound water and having the composition (in its anhydrous form) 0.7–1.1 $Na_2O \cdot Al_2O_3 \cdot 1.3–2.4\ SiO_2$ to 100 parts by weight polymer.

11. A stabilizer combination for polyvinyl chloride molding compositions, containing primary stabilizers selected from the group comprising metal soaps, aromatic metal carboxylates, metal phenolates and organotin compounds, costabilizers and, optionally, standard lubricants and

other standard processing aids, characterized in that they contain as costabilizers pyrazolinones corresponding to the following formula

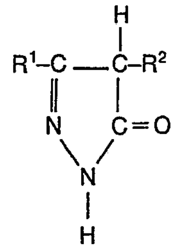

(I)

in which

$R^1$ and $R^2$ are linear $C_6$–$C_{11}$ alkyl radicals.

12. A stabilizer combination as claimed in Claim 11, characterized in that they contain pyrazolinones of formula I in which $R^1$ is an alkyl radical of the formula $C_nH_{2n+1}$ (II) and $R^2$ is an alkyl radical of the formula $C_{n-1}H_{2n-1}$ (III), in which formulae n may assume the values 7, 9 and 11.

13. A stabilizer combination as claimed in Claims 11 and 12, characterized in that they contain from 0.5 to 10 parts by weight calcium soaps and/or from 0.5 to 10 parts by weight barium soaps and/or from 0.5 to 10 parts by weight zinc soaps to 1 part by weight pyrazolinone of formula I.

14. A stabilizer combination as claimed in Claims 11 and 12, characterized in that it contains from 0.2 to 20 parts by weight of a synthetic, crystalline, finely divided sodium alumosilicate containing from 13 to 25% by weight bound water and having the composition (in its anhydrous form) 0.7–1.1 $Na_2O{\cdot}Al_2O_3{\cdot}1.3\text{–}2.4\ SiO_2$ to 1 part by weight pyrazolinone of formula I.

**Revendications**

1. Pyrazolinone répondant à la formule I

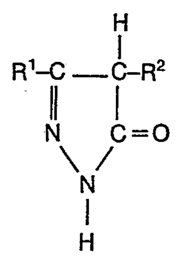

(I)

où $R^1$ et $R^2$ représentent des restes alcoyl à chaîne droite contenant 6 à 11 atomes de carbone.

2. Pyrazolinone suivant la revendication 1 où, dans la formule I, $R^1$ représente un reste alcoyl de formule $C_nH_{2n+1}$ (II), et $R^2$ un reste alcoyl de formule $C_{n-1}H_{2n-1}$ (III) et n peut prendre les valeurs 7, 9 et 11.

3. Procédé pour la fabrication de pyrazolinones de formule I

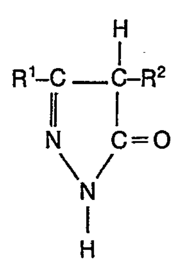

(I)

où $R^1$ et $R^2$ représentent des restes alcoyl à chaîne droite, à 6 à 11 atomes de carbone dans lequel on fait réagir un ester de l'acide β-cétocarboxylique de formule IV

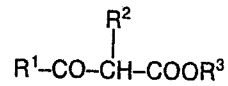

(IV)

où $R^1$ et $R^2$ représentent des restes alcoyl à chaîne droite avec 6 à 11 atomes de carbone, et $R^3$ un reste alcoyl avec 1 à 3 atomes de carbone, avec de l'hydrate d'hydrazine, et isole le produit recherché du mélange réactionnel.

4. Procédé suivant la revendication 3, caractérisé en ce que, dans la formule IV, $R^1$ est un reste alcoyl de formule $C_nH_{2n+1}$ (II) et $R^2$ un reste alcoyl de formule $C_{n-1}H_{2n-1}$ (III), n pouvant prendre les valeurs 7, 9 et 11.

5. Masse moulée en chlorure de polyvinyle stabilisé, contenant des pyrazolinones de formule I

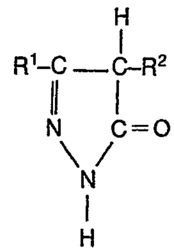

(I)

où $R^1$ et $R^2$ représentent des restes alcoyl à chaîne droite avec 6 à 11 atomes de carbone.

6. Masse moulées en chlorure de polyvinyle stabilisé suivant la revendication 5, contenant un agent lubrifiant courant et d'autres produits auxiliaires de transformation courants, ainsi qu'une combinaison stabilisante de (1) des stabilisants primaires choisis dans le groupe constitué par les savons métalliques, les carboxylates aromatiques de métaux, les phénolates de métaux et les composés organiques de l'étain, et (2), des constabilisants, caractérisées en ce qu'elles contiennent, comme co-stabilisants, des pyrazolinones de la formule I de la revendication 1.

7. Masses moulées en chlorure de polyvinyle stabilisés suivant les revendication 5 et 6, caractérisées en ce qu'elles contiennent, pour 100 parties en poids de polymère 0,02 à 5 parties en poids de pyrazolinones.

8. Masses moulées en chlorure de polyvinyle stabilisé suivant les revendications 5 à 7, caractérisées en ce qu'elles contiennent des pyrazolinones de formule I, dans lesquelles $R^1$ est un reste alcoyl de formule $C_nH_{2n+1}$ (II), et $R^2$ un reste alcoyl de formule $C_{n-1}H_{2n-1}$ (III), n pouvant prendre les valeurs 7, 9 et 11.

9. Masses moulées en chlorure de polyvinyle stabilisé suivant les revendications 5 à 8, caractérisées en ce qu'elles contiennent pour 100 parties de poids de polymère 0,1 à 3 parties en poids de savon de calcium et/ou 0,1 à 3% en poids de savon de baryum, et/ou 0,1 à 3% en poids de savon de zinc, ces savons dérivant d'acides gras avec 8 à 22 atomes de carbone.

10. Masses moulées en chlorure de polyvinyle stabilisé suivant les revendications 5 à 9, caractérisées en ce qu'elles contiennent, pour 100 parties en poids de polymère, 0,2 à 5 parties en poids d'un alumosilicate de sodium synthétique, cristallin, finement dispersé, contenant 13 à 25% en poids d'eau combinée qui présente la composition, calculée sur la forme anhydre, 0,7 à 1,1 $Na_2O{\cdot}Al_2O_3{\cdot}1{,}3$ à 2,4 $SiO_2$.

11. Combinaison stabilisante pour masses moulées en chlorure de polyvinyle, comprenant des stabilisants primaires choisis dans le groupe constitué par les savons métalliques, les carboxylates métalliques aromatiques, les phénolates métalliques, et les composés organiques de l'étain, des costabilisants et éventuellement un lubrifiant courant ainsi que d'autres agents auxiliaires de transformation courants, combinaison caractérisée en ce qu'elle contient, comme costabilisants, des pyrazolinones de formule I

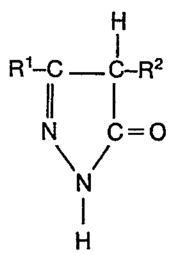

(I)

où $R^1$ et $R^2$ représentent des restes alcoyl à chaîne droite avec 6 à 11 atomes de carbone.

12. Combinaison stabilisante suivant la revendication 11, caractérisée en ce qu'elle contient des pyrazolinones de formule I, où $R^1$ est un reste alcoyl de formule $C_nH_{2n+1}$ (II), et $R^2$ un reste alcoyl de formule $C_{n-1}H_{2n-1}$ (III), n pouvant prendre les valeurs 7, 9 ou 11.

13. Combinaison stabilisante suivant les revendications 11 et 12, caractérisée en ce qu'elle contient, pour 1 partie en poids de pyrazolinone de formule I, 0,5 à 10 parties en poids de savon de calcium, et/ou 0,5 à 10 parties en poids de savon de baryum et/ou 0,5 à 10 parties en poids de savon de zinc, ces savons étant dérivés d'acides gras à 8 à 22 atomes de carbone.

14. Combinaison stabilisante suivant les revendications 11 et 12, caractérisée en ce qu'elle contient, pour 1 partie en poids de pyrazolinone de formule I, 0,2 à 20% en poids d'un alumosilicate de sodium synthétique, cristallin, finement dispersé, contenant 13 à 25% en poids d'eau combinée et qui a la composition suivante, calculée sur la forme anhydre: 0,7 à 1,1 $Na_2O{\cdot}Al_2O_3{\cdot}1{,}3$ à 2,4 $SiO_2$.